# EUROPÄISCHE PATENTSCHRIFT

(11) **EP 0 700 892 B1**
(45) Veröffentlichungstag und Bekanntmachung des Hinweises auf die Patenterteilung: **24.03.1999**
(21) Anmeldenummer: 95113216.6
(22) Anmeldetag: 23.08.1995
(51) Int. Cl.: C07C 51/235, C07C 55/02, C07C 53/126

(54) **Verfahren zur Herstellung von Mono- oder Dicarbonsäuren aus Aldehyden, deren Vollacetalen oder Halbacetalen, sowie aus Gemischen davon**
Process for preparing mono- or dicarboxylic acids from aldehydes, their acetals or hemi-acetals as well as from their mixtures
Procédé de préparation d'acides mono- ou dicarboxyliques à partir des aldéhydes, leurs acétals ou hemi-acétals, comme aussi à partir de leurs mélanges

(30) Priorität: 06.09.1994 AT 1701/94
(43) Veröffentlichungstag der Anmeldung: 13.03.1996
(73) Patentinhaber: DSM Chemie Linz GmbH, 4021 Linz (AT)
(72) Erfinder: Kos, Carlo, Dr., A-4060 Leonding (AT); Schöftner, Manfred, A-4020 Linz (AT); Friedhuber, Johann, A-4030 Linz (AT)
(74) Vertreter: Kunz, Ekkehard, Dr.

(56) Entgegenhaltungen:
- EP-A- 0 147 593
- DE-A- 1 668 485
- GB-A- 955 421
- US-A- 2 010 358
- US-A- 3 415 877

## Beschreibung

Mono- und Dicarbonsäuren, insbesondere alpha-omega-Alkandicarbonsäuren, sind wertvolle Ausgangsprodukte in der chemischen Industrie und finden beispielsweise bei der Herstellung von Pharmazeutika, Kosmetika, Schmiermittel und dgl. Verwendung.
Aus der Literatur ist daher eine Vielzahl von Herstellungsmöglichkeiten für Mono- und Dicarbonsäuren bekannt.
Beispielsweise ist in GB 2,034,310 die Oxidation von Aldehydsäuren bzw. Dialdehyden in saurer Lösung und in Gegenwart eines Kobalt-Salz und/oder eines Eisen-Salz-Katalysators beschrieben.
Auch nach GB 1,539,573 ist zur Oxidation von Aldehydsäuren zu den entsprechenden Dicarbonsäuren ein Katalysator notwendig.
Der Nachteil aller bisher beschriebenen Oxidationen in saurer Lösung ist jedoch die Notwendigkeit eines Katalysators, der nach erfolgter Reaktion aus dem Reaktionsgemisch entfernt werden muß.
Verfahren, die ohne Zusatz eines Katalysators durchgeführt werden, sind ebenfalls bekannt, weisen jedoch den Nachteil auf, daß die Oxidation in aprotischen Lösungsmitteln, wie etwa Chlorbenzol, stattfindet. Ein weiterer Nachteil ist, daß bei diesen Verfahren die Aldehyde keinen Acetalanteil aufweisen dürfen.

Aus US 2,010,358 ist weiters ein Verfahren zur Oxidation von Aldehyden, die ein tertiäres C-Atom anschließend an die Aldehydgruppe aufweisen, beschrieben. Die Reaktionstemperatur liegt dabei unter 45°C um eine oxidative Zersetzung der Aldehyde zu verhindern. Als Verdünnungsmittel dient die hergestellte Carbonsäure selbst oder eine andere Carbonsäure. Der Wassergehalt sollte jedoch 5 % nicht übersteigen. Die Reaktion kann gemäß der Beschreibung sowohl mit als auch ohne Katalysator erfolgen, gemäß den Beispielen erfolgt sie jedoch immer in Anwesenheit eines Katalysators. Die Möglichkeit, daß diese Aldehyde einen Acetalanteil aufweisen können, ist aus US 2,010,358 nicht ersichtlich.

Aufgabe der vorliegenden Erfindung war es demnach, ein Verfahren zu finden, das Mono- und Dicarbonsäuren in hoher Ausbeute und Reinheit ohne Einsatz eines Katalysators und ohne die Verwendung eines aprotischen Lösungsmittels liefert, wobei die Ausgangsprodukte auch einen Acetalanteil aufweisen können.

Gegenstand der vorliegenden Erfindung ist demnach ein Verfahren zur Herstellung von aliphatischen oder aromatischen Mono- oder Dicarbonsäuren mit 4 - 22 C-Atomen durch Oxidation der entsprechenden Aldehyde mit der gleichen Zahl Kohlenstoffatome, das dadurch gekennzeichnet ist, daß als Aldehyd ein aliphatischer oder aromatischer Aldehyd mit ein oder zwei Aldehydgruppen, Halbacetal- oder Vollacetalgruppen oder Gemische derselben eingesetzt und daß die Oxidation in Abwesenheit eines Katalysators in einer Carbonsäure aus der Gruppe Ameisensäure, Essigsäure und Propionsäure, oder in einem Carbonsäure-Wasser-Gemisch bei einer Temperatur von 50 - 130°C und bei 1 - 25 bar in Gegenwart eines Oxidationsmittels durchgeführt wird.
Nach dem erfindungsgemäßen Verfahren werden Mono- oder Dicarbonsäuren durch Oxidation der entsprechenden Aldehyde gewonnen.
Als Aldehyde kommen dabei ein Aldehyd selbst, dessen Voll- oder Halbacetale oder ein Gemisch aus Aldehyd und dessen Halbacetale und/oder Vollacetale in Frage. Geeignete Gemische aus Aldehyd und Acetalen können zwischen 1 - 99 % Aldehydanteil bzw. 1 - 99 % Acetalanteil beinhalten.
Die eingesetzten Aldehyde können daher sowohl aliphatische, als auch aromatische Aldehyde mit jeweils einer oder zwei Aldehydgruppen bzw. Halbacetal- oder Vollacetal-Gruppen sein. Ein aliphatischer Aldehyd ist ein geradkettiger, verzweigter oder cyclischer Mono- oder Dialdehyd mit 4 bis 22 C-Atomen, bevorzugt mit 6 bis 15 C-Atomen, wobei die Alkylketten unsubstituiert oder durch unter den Reaktionsbedingungen inerte Gruppen substituiert sein können. Solche inerte Gruppen sind Beispielsweise durch Alkyl, bevorzugt mit 1 bis 6, besonders bevorzugt mit 1 bis 4 C-Atomen, wie Methyl, Ethyl, iso-Propyl, Butyl, neo-Pentyl, oder Hexyl, Alkoxy, bevorzugt mit 1 bis 6, besonders bevorzugt mit 1 bis 4 C-Atomen, wie Methoxy, Ethoxy-, Butoxy-, iso-Pentoxy- oder Hexoxygruppen, oder durch Halogen substituiertes Phenyl oder Naphthyl, oder Alkoxygruppen, bevorzugt solche mit 1 bis 6 C-Atomen. Weitere Beispiele für unter den Reaktionsbedingungen inerte Gruppen sind Estergruppen, bevorzugt mit 1 - 4 C-Atomen im Esterteil.

Aromatische Aldehyde sind Aldehyde, bei denen eine oder zwei Aldehydgruppen direkt an ein aromatisches C-Atom gebunden sind, beispielsweise in einer Phenyl-, Naphthyl-, oder Pyridingruppe wie Benzaldehyd, Phthalaldehyd, Naphthylaldehyde, Pyridinaldehyd- oder -dialdehyde.
Bevorzugt werden unsubstituierte aliphatische Mono- und Dialdehyde, besonders bevorzugt Dialdehyde, mit 4 bis 22 C-Atomen eingesetzt.
Zur Herstellung von Aldehyden ist eine Vielzahl von Verfahren bekannt. Gemäß US 4 607 126 oder US 4 769 464 können Aldehyde oder Dialdehyde in technischem Maßstab auf bequeme Weise, beispielsweise durch Ozonolyse und Reduktion olefinischer Doppelbindungen hergestellt werden.

Zur Durchführung der Reaktion gemäß US 4 769 464 zur Herstellung der Aldehyde wird eine entsprechende chemische Verbindung mit mindestens einer olefinischen Doppelbindung in einem organischen Lösungsmittel, in dem sie gut löslich ist, beispielsweise in einem niedrigen aliphatischen Alkohol, vorzugsweise in Methanol gelöst und mit der äquivalenten Menge Ozon behandelt. Die Ozonisierung wird bei Temperaturen von etwa -30 bis 0°C, vorzugsweise etwa -20 bis 0°C durchgeführt.

Die an die Ozonisierung anschließende katalytische Hydrierung der Ozonolyseprodukte wird in verdünnter Lösung durchgeführt, wobei vorzugweise während der Hydrierung ein kontrollierter Peroxidgehalt von höchstens 0,1 mol/l eingehalten wird. Dazu wird eine Suspension des Katalysators im Lösungsmittel und Wasserstoff vorgelegt und die Ozonolyselösung kontinuierlich eingespeist. Durch den durch diese Maßnahme niedrig gehaltenen Peroxidgehalt des Reaktionsmediums wird eine Vergiftung und ein Aktivitätsverlust des Katalysators verhindert.

Die Hydrierung erfolgt unter praktisch drucklosen Bedingungen, das sind Drücke von 1 bis 3 bar, die üblicherweise angewendet werden, um das Eindringen von Luft in den Hydrierreaktor zu verhindern.
Die Reaktionstemperatur bei der Hydrierung beträgt etwa 20 - 40°C. Der pH-Wert wird während der Hydrierung in einem Bereich von 2 - 7 gehalten. Da während der Hydrierung geringe Mengen an sauren Nebenprodukten entstehen können, kann der pH-Wert gegebenenfalls durch Zugabe einer Base, vorzugsweise von verdünnter Natronlauge oder Kalilauge im gewünschten Bereich gehalten werden.
Je nach pH-Wert Bedingungen entstehen dabei Aldehyde, deren Halbacetale, deren Vollacetale oder Mischungen derselben. Zur Isolierung der Aldehyde wird der Katalysator abfiltriert und das Lösungsmittel entfernt.
Aldehyde die gemäß dem beschriebenen Verfahren herstellbar sind, werden bevorzugt in dem erfindungsgemäßen Verfahren eingesetzt.

Der entsprechende Aldehyd wird in einer Carbonsäure als Verdünnungsmittel oder in einem Carbonsäure-Wasser-Gemisch mit Hilfe eines Oxidationsmittels zu der gewünschten Mono- oder Dicarbonsäure oxidiert. Als Verdünnungsmittel geeignete Carbonsäuren sind dabei Ameisensäure, Essigsäure, Propionsäure und dergleichen. Bevorzugt wird Essigsäure als Lösungsmittel eingesetzt. Die Carbonsäuren können entweder alleine, oder im Gemisch mit Wasser eingesetzt werden. Bevorzugt werden die Carbonsäuren im Gemisch mit Wasser verwendet. Der Anteil an Wasser kann dabei etwa 0,1 - 25 Vol%, bevorzugt 5 - 15 Vol%, betragen.
Die Konzentration der Aldehyde in der Lösung ist für das erfindungsgemäße Verfahren nur von geringer Bedeutung. Im allgemeinen werden möglichst hohe Aldehydkonzentrationen hergestellt um Lösungsmittel zu sparen, wobei die Konzentration aber nicht so hoch sein soll, daß die Aldehyde oder die gebildeten Carbonsäuren bei Reaktionstemperatur aus der Reaktionslösung ausfallen.
Als Oxidationsmittel wird Sauerstoff oder Luft, vorzugsweise Sauerstoff eingesetzt.

Die Oxidation wird vorzugsweise unter Druck durchgeführt, wobei Drücke von 1 bis 25 bar, bevorzugt von 8 bis 25 bar, angewandt werden. Die Reaktionstemperatur beträgt etwa 50 bis 130°C, bevorzugt 60 bis 90°C.
Nach beendeter Reaktion wird die Reaktionslösung abgekühlt, bevorzugt auf 0° bis 35°C und je nach Reaktionsprodukt entsprechend aufgearbeitet.
Thermisch stabile Monocarbonsäuren werden beispielsweise destillativ abgetrennt, thermisch unstabile Monocarbonsäuren werden hingegen, sofern sie wasserunlöslich sind, durch Auswaschen des Lösungsmittels mit Wasser aus dem Reaktionsgemisch isoliert. Dicarbonsäuren werden beispielsweise, soweit sie in der Kälte, bei 0 - 35°C, im Lösungsmittel schlecht oder unlöslich sind, durch Kristallisation und anschließender Feststofftrennung isoliert. Die dabei anfallende Mutterlauge kann, nach Ergänzen mit der Aldehydkomponente und Wasser wieder direkt dem Oxidationsprozeß zugeführt werden. Die so isolierten Carbonsäuren können gewünschtenfalls noch durch übliche Verfahren, etwa Umkristallisieren oder Destillation bzw. chromatographische Methoden gereinigt werden. Das erfindungsgemäße Verfahren kann sowohl diskontinuierlich als auch kontinuierlich durchgeführt werden.

In einer bevorzugten Ausführungsform des erfindungsgemäßen Verfahrens werden aliphatische und aromatische Mono- oder Dicarbonsäuren mit 4 bis 22 C-Atomen dadurch hergestellt, daß eine entsprechende chemische Verbindung mit mindestens einer olefinischen Doppelbindung mit Ozon in Gegenwart eines Lösungsmittels umgesetzt, die entstandene Peroxydlösung katalytisch hydriert wird, worauf der entstandene Aldehyd, nach Abtrennung des Lösungsmittels und des Katalysators, in Gegenwart einer Carbonsäure, aus der Gruppe Ameisensäure, Essigsäure und Propionsäure, oder eines Carbonsäure-Wasser Gemisches ohne Katalysator zur gewünschten Mono- oder Dicarbonsäure oxidiert wird.

Die Ozonisierung und anschließende Hydrierung wird dabei vorzugsweise nach dem in US 4 769 464 beschriebenen Verfahren, die Oxidation wird unter den oben angeführten Bedingungen durchgeführt. Als chemische Verbindung mit mindestens einer olefinischen Doppelbindung kommen dabei solche Verbindungen in Frage, die zu den oben angeführten Aldehyden führen.

Beispiele dafür sind geradkettige, verzweigte oder cyclische (C₄ - C₂₂) Alkene mit mindestens einer olefinischen Doppelbindung, die gegebenenfalls durch die bereits erwähnten unter den Reaktionsbedingungen inerten Gruppen substituiert sein können, wie etwa Buten, Penten, Hexen, Isopren, Isobuten, Isoocten, Butadien, Oktadien, Hexadecen, Cyclohexen, Cyclooctadien, Cycloocten, Cyclododecen, Cyclododecatrien, Terpene oder aromatische Verbindungen mit mindestens einer olefinischen Doppelbindung, wie beispielsweise Styrole, Divinylbenzole, Diisopropenylbenzol, Naphthylstyrole oder Diphenylethylene.
Nach dem erfindungsgemäßen Verfahren werden Mono- und Dicarbonsäuren in hoher Reinheit und mit ausgezeichneten Ausbeuten erhalten. In der Regel werden Ausbeuten von 90 - 98 % ohne wesentliche Abfallprodukte oder Abwässer und umweltbelastenden Abgasen erzielt. Die Mono- und Dicarbonsäuren werden mit mindestens 95 %iger Reinheit, üblicherweise aber mit über 98 %iger Reinheit, erhalten, wodurch eine weitere Reinigung des Endproduktes in den meisten Fällen nicht nötig ist.

### Beispiel 1: Oktandisäure

110,2 g (1 mol) Cycloocten (Reinheit 95 %) wurden in 1500 ml Methanol gelöst, auf -20°C gekühlt und ein O₂/O₃-Gemisch, das 4 Gew.% Ozon enthielt, eingeleitet bis 1 mol Ozon in die Lösung eingebracht worden ist.
Anschließend wurde in einem Hydrierreaktor, in dem 2 g Pd-Katalysator (Lindlar) vorgelegt wurden und der mit Wasserstoff gefüllt wurde, die erhaltene Ozonisierungslösung über ein Dosiergefäß kontinuierlich eingespeist, daß der Peroxidgehalt 0,02 mol/l nicht überstieg.
Unter kräftigem Rühren und Wasserstoffzugabe wurde bis zur negativen Peroxidprobe hydriert.

Aus der Hydrierlösung wurde der Katalysator abfiltriert, das Lösungsmittel unter Vakuum entfernt und der Rückstand in 90 %iger Essigsäure gelöst, sodaß die Konzentration des Aldehyds 1 mol/l Lösung betrug.
Die Lösung wurde in einem Autoklaven mit Sauerstoff bei einem Druck von 10 bar und einer Temperatur von 80°C oxidiert. Nach beendeter Reaktion wurde der Autoklav entspannt und die Lösung auf 15 - 20°C abgekühlt. Der entstandene Niederschlag wurde abfiltriert und im Vakuum von 20 mbar bei 80°C getrocknet. Die Mutterlauge wurde mit Wasser, Lösungsmittel und Rohaldehyd auf die geforderte Konzentration gebracht und im Kreislauf wieder in der Oxidationsstufe eingesetzt. Es wurden so 125 - 152 g Oktandisäure pro Cyclus mit einer Reinheit >98 % erhalten.

### Beispiel 2: Oktandisäure

Analog Beispiel 1 wurden 108,18 g (1 mol) Cyclooctadien (Reinheit 95 %) mit Ozon versetzt, bis 0,5 mol Ozon in die Lösung eingebracht wurden, und die so erhaltene Ozonisierungslösung analog Beispiel 1 hydriert.
Die so erhaltene Hydrierlösung enthielt Oktandial und dessen Acetal.

Die Oxidation des Aldehyd/Acetal-Gemisches wurde analog Beispiel 1 durchgeführt.
Es wurden so 125 g Octandisäure pro Cyclus mit einer Reinheit von 96 % erhalten.

### Beispiel 3: Dodecandisäure

Analog Beispiel 1 wurden 166,4 g (1 mol) Cyclododecen (Reinheit 95 %) zu Dodecandial ozonisiert und anschließend katalytisch hydriert.
Die Oxidation von Dodecandial erfolgte ebenfalls analog Beispiel 1.
Es wurden 220 g Dodecandisäure pro Cyclus mit einer Reinheit von 98 % erhalten.

### Beispiel 4: Dodecandisäure

162,3 g (1 mol) Cyclododecatrien (Reinheit 95 %) wurden in 1500 ml Methanol gelöst auf -20°C gekühlt und ein O₃/O₂-Gemisch das 4 Gew.% O₃ enthielt eingeleitet bis 0,33 mol Ozon in die Lösung eingebracht worden sind. Die analog Beispiel 1 erhaltene Hydrierlösung enthielt Dodecandial und dessen Acetale.
Entstandenes Cyclododecan wurde mit Petrolether aus der Essigsäurelösung vor der Oxidation extraktiv entfernt.

Die Oxidation des Aldehyd/Acetal-Gemisches wurde analog Beispiel 1 bei 15 bar durchgeführt
Es wurden 183 g Dodecandisäure pro Cyclus mit einer Reinheit von 98 % erhalten.

### Beispiel 5: Tridecandisäure

338,58 g (1 mol) Erukasäure (Reinheit 95 %) wurden analog Beispiel 1 ozonisiert und anschließend hydriert.
Die so erhaltene Hydrierlösung enthielt Nonanal und Tridecanaldehydsäure und wurde destillativ aufgetrennt.
Zur Oxidation wurde die entsprechende Aldehydfraktion eingesetzt und analog Beispiel 1 bei 14 bar oxidiert. Es wurden 185 g Tridecandisäure pro Cyclus mit einer Reinheit von 95 % erhalten.

### Beispiel 6: Pelargonsäure

338,58 g (1 mol) Erukasäure (Reinheit 93 %) wurden analog Beispiel 1 ozonisiert und anschließend hydriert. Die so erhaltene Hydrierlösung enthielt Pelargonaldehyd und Tridecanalsäure.
Zur Oxidation wurde die entsprechende Aldehydfraktion eingesetzt und analog Beispiel 1 bei 20 bar oxidiert.
Es wurden 132 g Pelargonsäure pro Cyclus mit einer Reinheit von 94 % erhalten.

### Beispiel 7: Pentadecansäure

224,4 g (1 mol) Hexadecen (Reinheit 95 %) wurden analog Beispiel 1 ozonisiert und anschließend zu Pentadecanal hydriert.

Die Oxidation des Aldehyds erfolgte analog Beispiel 1.
Nach beendeter Reaktion wurde der Autoklav entspannt, das Lösungsmittel im Vakuum entfernt und der Rückstand unter Kühlung im Wasser gerührt.
Die so erhaltene Pentadecansäure wird abgetrennt und getrocknet.
Ausbeute: 92 %
Reinheit: 93 %

Weiters wurden nach dem erfindungsgemäßen Verfahren folgende Produkte hergestellt:

| Bsp. | E | P | p (bar) | T (°C) | A (%) | R (%) |
|---|---|---|---|---|---|---|
| 8 | 13-Oxotridecansäuremethylester | Brassylsäuremonomethylester | 14 | 64-90 | 92 | 95 |
| 9 | 2-Ethylhexanal | 2-Ethylhexansäure | 20 | 80 | 98 | 95 |
| 10 | 1,12-Dimethoxy-1,12-dihydroxydodecan | Dodecandisäure | 16 | 80-85 | 99 | 95 |
| 11 | 1,1,8,8-Tetramethoxyoctan | Oktandisäure | 10 | 80-85 | 90 | 90 |
| 12 | Heptandialdehyd | Heptandisäure | 15 | 80-85 | 97 | 90 |
| 13 | 9-Oxo-Nonansäuremethylester | Azelainsäuremonomethylester | 10 | 80-83 | 92 | 94 |
| 14 | Benzaldehyd | Benzoesäure | 12 | 80-85 | 98 | 98 |
| Als Verdünnungsmittel wurde jeweils Essigsäure eingesetzt. Es wurden folgende Abkürzungen verwendet: E Edukt für die Oxidation P Produkt, erhalten durch Oxidation p Druck T Temperatur A Ausbeute R Reinheit | | | | | | |

## Patentansprüche

1. Verfahren zur Herstellung von aliphatischen oder aromatischen Mono- oder Dicarbonsäuren mit 4 bis 22 C-Atomen durch Oxidation der entsprechenden Aldehyde mit gleicher Zahl Kohlenstoffatome, dadurch gekennzeichnet, daß als Aldehyd ein aliphatischer oder aromatischer Aldehyd mit ein oder zwei Aldehydgruppen, Halbacetal- oder Vollacetalgruppen oder Gemische derselben eingesetzt und daß die Oxidation in Abwesenheit eines Katalysators in einer Carbonsäure aus der Gruppe Ameisensäure, Essigsäure und Propionsäure, oder in einem Carbonsäure-Wasser-Gemisch bei einer Temperatur von 50 - 130°C und 1 bis 25 bar in Gegenwart eines Oxidationsmittels durchgeführt wird.

2. Verfahren nach Anspruch 1, dadurch gekennzeichnet, daß ein Gemisch aus Aldehyd und dessen Voll- und/oder Halbacetalen, wobei der Aldehydanteil zwischen 1 - 99 % betragen kann, eingesetzt wird.

3. Verfahren nach Anspruch 1, dadurch gekennzeichnet, daß als Carbonsäure Essigsäure verwendet wird.

4. Verfahren nach Anspruch 1, dadurch gekennzeichnet, daß die Oxidation in einem Carbonsäure-Wasser Gemisch, mit 0,1 bis 25 Vol % Wasseranteil durchgeführt wird.

5. Verfahren nach Anspruch 1, dadurch gekennzeichnet, daß die Oxidation bei einer Temperatur von 60 bis 90°C durchgeführt wird.

6. Verfahren nach Anspruch 1, dadurch gekennzeichnet, daß die Oxidation bei 8 bis 25 bar durchgeführt wird.

7. Verfahren nach Anspruch 1, dadurch gekennzeichnet, daß als Oxidationsmittel Sauerstoff verwendet wird.

8. Verfahren nach Anspruch 1, dadurch gekennzeichnet, daß eine chemische Verbindung mit mindestens einer olefinischen Doppelbindung mit Ozon in Gegenwart eines Lösungsmittels umgesetzt, die entstandene Peroxidlösung katalytisch hydriert wird, worauf der entstandene Aldehyd nach Abtrennung des Lösungsmittels und des Katalysators, in Gegenwart einer Carbonsäure aus der Gruppe Essigsäure, Ameisensäure und Propionsäure oder eines Carbonsäure-Wasser Gemisches bei 50 - 130°C und 1 - 25 bar in Gegenwart eines Oxidationsmittels zur gewünschten Mono- oder Dicarbonsäure oxidiert wird.

## Claims

1. Process for the preparation of aliphatic or aromatic mono- or dicarboxylic acids of 4 to 22 carbon atoms by oxidation of the corresponding aldehydes having the same number of carbon atoms, characterized in that an aliphatic or aromatic aldehyde having one or two aldehyde groups, hemiacetal or full acetal groups, or mixtures thereof, is employed as aldehyde and in that the oxidation is carried out in the absence of a catalyst in a carboxylic acid from the group consisting of formic acid, acetic acid and propionic acid, or in a carboxylic acid/water mixture, at a temperature of 50 - 130°C and at from 1 to 25 bar in the presence of an oxidizing agent.

2. Process according to Claim 1, characterized in that a mixture of aldehyde and its full acetals and/or hemiacetals, in which the aldehyde content can be 1 - 99%, is used.

3. Process according to Claim 1, characterized in that acetic acid is used as carboxylic acid.

4. Process according to Claim 1, characterized in that the oxidation is carried out in a carboxylic acid/ water mixture with a water content of from 0.1 to 25% by volume.

5. Process according to Claim 1, characterized in that the oxidation is carried out at a temperature of from 60 to 90°C.

6. Process according to Claim 1, characterized in that the oxidation is carried out at from 8 to 25 bar.

7. Process according to Claim 1, characterized in that oxygen is used as oxidizing agent.

8. Process according to Claim 1, characterized in that a chemical compound having at least one olefinic double bond is reacted with ozone in the presence of a solvent, the resulting peroxide solution is catalytically hydrogenated, and then the resulting aldehyde, after separating off the solvent and the catalyst, is oxidized in the presence of a carboxylic acid from the group consisting of acetic acid, formic acid and propionic acid, or of a carboxylic acid/water mixture, at 50 - 130°C and 1 - 25 bar, in the presence of an oxidizing agent, to give the desired mono- or dicarboxylic acid.

## Revendications

1. Procédé de préparation d'acides mono- ou dicarboxyliques aliphatiques ou aromatiques ayant de 4 à 22 atomes de carbone, par oxydation des aldéhydes correspondants ayant le même nombre d'atomes de carbone, caractérisé en ce qu'on utilise, comme aldéhyde, un aldéhyde aliphatique ou aromatique ayant un ou deux groupes aldéhydes, hémi-acétals ou acétals, ou des mélanges de ceux-ci, et en ce qu'on effectue l'oxydation en l'absence de catalyseur dans un acide carboxylique choisi dans l'ensemble constitué par l'acide formique, l'acide acétique et l'acide propionique, ou dans un mélange d'acide carboxylique et d'eau, à une température de 50 à 130°C et à une pression de 1 à 25 bars, en présence d'un agent oxydant.

2. Procédé selon la revendication 1, caractérisé en ce qu'on utilise un mélange d'aldéhyde et de ses acétals et/ou hémi-acétals, la proportion d'aldéhyde pouvant être comprise entre 1 et 99 %.

3. Procédé selon la revendication 1, caractérisé en ce qu'on utilise de l'acide acétique comme acide carboxylique.

4. Procédé selon la revendication 1, caractérisé en ce qu'on effectue l'oxydation dans un mélange d'acide carboxylique et d'eau avec une proportion d'eau de 0,1 à 25 % en volume.

5. Procédé selon la revendication 1, caractérisé en ce qu'on effectue l'oxydation à une température de 60 à 90°C.

6. Procédé selon la revendication 1, caractérisé en ce qu'on effectue l'oxydation à une pression de 8 à 25 bars.

7. Procédé selon la revendication 1, caractérisé en ce qu'on utilise de l'oxygène comme agent oxydant.

8. Procédé selon la revendication 1, caractérisé en ce qu'on fait réagir un composé chimique ayant au moins une liaison double oléfinique avec de l'ozone en présence d'un solvant, on hydrogène catalytiquement la solution de peroxyde formée, après quoi, après séparation du solvant et du catalyseur, on oxyde l'aldéhyde formé, en présence d'un acide carboxylique choisi dans l'ensemble constitué par l'acide acétique, l'acide formique et l'acide propionique, ou d'un mélange d'acide carboxylique et d'eau, à une température de 50 à 130°C et à une pression de 1 à 25 bars, en présence d'un agent oxydant, pour obtenir l'acide mono- ou dicarboxylique souhaité.
